# EUROPEAN PATENT APPLICATION

(11) **EP 4 825 819 A1**
(43) Date of publication of application: **21.10.2026**
(21) Application number: 25738739.9
(22) Date of filing: 06.01.2025
(51) Int. Cl.: C03C 17/32, B32B 17/10, B32B 27/30, C03C 17/34, C08F 20/28, C12M 1/00, C12M 1/26

(54) **POLYMER-COATED GLASS BASE MATERIAL**

(30) Priority: 12.01.2024 JP 2024003208; 21.11.2024 JP 2024203197
(71) Applicant: SUMITOMO RUBBER INDUSTRIES, LTD., Kobe-shi, Hyogo 651-0072 (JP)
(72) Inventor: MINAGAWA, Yasuhisa, Kobe-shi, Hyogo 651-0072 (JP)
(74) Representative: Manitz Finsterwald Patent- und Rechtsanwaltspartnerschaft mbB
(86) International application number: PCT/JP2025/000031
(87) International publication number: WO 2025/150470

(57) **Abstract**

The present invention provides a polymer-coated glass substrate capable of inhibiting the adsorption of normal cells such as blood cells and achieving selective adsorption of specific cells such as cancer cells, stem cells, or T cells. The present invention relates to a polymer-coated glass substrate, including: a glass substrate; and one or two or more polymer layers formed on a surface of the glass substrate, the glass being a soda-lime glass with a bubble contact angle in water of 100° or greater and 120° or less, an outermost layer among the one or two or more polymer layers having a surface with an elastic modulus in water or an aqueous solution of 1.20 MPa or lower.

## Description

### TECHNICAL FIELD

The present invention relates to polymer-coated glass substrates.

### BACKGROUND ART

A technique of coating the surface of a substrate with a special polymer has been proposed to produce a device for capturing specific cells (e.g., blood cells and cells in blood or biological fluid such as cancer cells, stem cells, and T-cells) from blood or biological fluid.

However, blood cells may simultaneously adsorb when specific cells such as cancer cells, stem cells, or T cells adsorb to the surface of the substrate. Thus, it is desirable to provide a polymer-coated substrate capable of inhibiting the adsorption of normal cells such as blood cells while achieving selective adsorption of specific cells such as cancer cells, stem cells, or T cells.

### SUMMARY OF INVENTION

### - Technical Problem

The present invention aims to solve the above problem and provide a polymer-coated glass substrate capable of inhibiting the adsorption of normal cells such as blood cells and achieving selective adsorption of specific cells such as cancer cells, stem cells, or T cells.

### - Solution to Problem

The present invention relates to a polymer-coated glass substrate, including: a glass substrate; and one or two or more polymer layers formed on a surface of the glass substrate, the glass being a soda-lime glass with a bubble contact angle in water of 100° or greater and 120° or less, an outermost layer among the one or two or more polymer layers having a surface with an elastic modulus in water or an aqueous solution of 1.20 MPa or lower.

### - Advantageous Effects of Invention

The polymer-coated glass substrate of the present invention includes: a glass substrate; and one or two or more polymer layers formed on a surface of the glass substrate, the glass being a soda-lime glass with a bubble contact angle in water of 100° or greater and 120° or less, an outermost layer among the one or two or more polymer layers having a surface with an elastic modulus in water or an aqueous solution of 1.20 MPa or lower. The polymer-coated glass substrate is capable of inhibiting the adsorption of normal cells such as blood cells and achieving selective adsorption of specific cells such as cancer cells, stem cells, or T cells. Thus, the polymer-coated glass substrate is expected to enhance the adsorption of specific cells such as cancer cells, stem cells, or T cells.

### DESCRIPTION OF EMBODIMENTS

The polymer-coated glass substrate includes a glass substrate and one or two or more polymer layers formed on a surface of the glass substrate. The glass is a soda-lime glass with a bubble contact angle in water of 100° or greater and 120° or less. An outermost layer among the one or two or more polymer layers has a surface with an elastic modulus in water or an aqueous solution of 1.20 MPa or lower.

The number of tumor cells (e.g., cancer cells) appearing in biological fluid, such as circulating tumor cells (several to hundreds of cells/1 ml of blood), is very small. In order to analyze tumor cells in a sampled biological fluid, it is considered important to increase adsorption of the tumor cells as much as possible. In the above-described polymer-coated glass substrate, a soda-lime glass with a bubble contact angle in water of 100° or greater and 120° or less is used as a glass substrate for covering polymers, and also the outermost layer among the one or two or more polymer layers formed on the substrate surface has a surface with an elastic modulus in water or an aqueous solution of 1.20 MPa or lower. Specific cells such as cancer cells are known to be generally softer than normal cells such as blood cells. This is related to the fact that when specific cells such as cancer cells metastasize, they deform their cell shape greatly and move through gaps. Normal cells such as blood cells, which are less deformable and harder, are less likely to be adsorbed to a polymer substrate having a soft surface. In contrast, specific cells such as cancer cells that have acquired deformability easily adsorb to even a polymer substrate having a soft surface. Yet, the adsorption decreases when the surface has an excessively low elastic modulus. In other words, the surface has an optimal elastic modulus. In particular, the glass substrate used in the polymer-coated glass substrate is a soda-lime glass with a bubble contact angle in water of 100° or greater and 120° or less. Moreover, one or two or more polymer layers are formed on the substrate, and the outermost layer among them has a surface with an elastic modulus in water or an aqueous solution of 1.20 MPa or lower. Such a polymer-coated glass substrate can exhibit a significant effect of achieving selective adsorption of specific cells such as cancer cells, stem cells, or T cells while inhibiting the adsorption of normal cells such as blood cells. It is expected that the number of tumor cells in the biological fluid may be determined from the count of tumor cells adsorbed to the polymer layer of the polymer-coated glass substrate, and the cancer-treating effect or the like may be evaluated based on the determined number. Moreover, the adsorbed tumor cells may be cultured and then used to determine the effects of drugs such as anticancer drugs ex vivo before administration of the drugs such as anticancer drugs. The adsorbed tumor cells can also be utilized to screen drugs such as anticancer drugs. Furthermore, genetic analysis of the adsorbed tumor cells helps to screen drugs such as anticancer drugs. Additionally, the adsorbed or cultured tumor cells may be subjected to genetic analysis to examine the mutation and expression of the tumor cells. The analysis result may be used to screen drugs such as anticancer cells or may be utilized to elucidate the mechanisms of cancers.

The glass constituting the glass substrate is a soda-lime glass.

With regard to the soda-lime glass, the soda-lime glass substrate may have a surface having at least one group represented by -N(R¹)₂ or -N(R²)₃⁺ wherein R¹s or R²s are the same as or different from each other and each represent a hydrogen atom or a substituted or unsubstituted hydrocarbon group optionally containing a heteroatom. In this case, the polymer-coated glass substrate tends to efficiently exhibit the effect of inhibiting the adsorption of normal cells such as blood cells and achieving selective adsorption of specific cells such as cancer cells, stem cells, or T cells.

With regard to "at least one group represented by -N(R¹)₂ or -N(R²)₃⁺", R¹s in the group represented by -N(R¹)₂ or R²s in the group represented by -N(R²)₃⁺ are the same as or different from each other and each represent 1) a hydrogen atom or 2) a substituted or unsubstituted hydrocarbon group optionally containing a heteroatom.

The substituted or unsubstituted hydrocarbon group optionally containing a heteroatom for R¹ and R² may be a linear, branched, or cyclic group. Non-limiting examples of the heteroatom include oxygen and nitrogen. Non-limiting examples of the substituent include known groups such as a hydroxy group and a halogen group (e.g., -Cl, -Br). The number of the heteroatom or the substituent may be one or two or more.

The number of carbon atoms in the substituted or unsubstituted hydrocarbon group optionally containing a heteroatom is preferably 1 to 20, more preferably 1 to 10, still more preferably 1 to 8.

Examples of the substituted or unsubstituted hydrocarbon group optionally containing a heteroatom include monovalent hydrocarbon groups and divalent hydrocarbon groups.

Examples of the substituted or unsubstituted monovalent hydrocarbon groups optionally containing a heteroatom include substituted or unsubstituted linear, branched, or cyclic alkyl, cyclic ether, aryl, aralkyl, and alkoxy groups each optionally containing a heteroatom.

Examples of the substituted or unsubstituted linear or branched alkyl groups each optionally containing a heteroatom include a methyl group, an ethyl group, an n-propyl group, an isopropyl group, an n-butyl group, an iso-butyl group, a sec-butyl group, a tert-butyl group, a pentyl group, a hexyl group, a heptyl group, a 2-ethylhexyl group, an octyl group, a nonyl group, a decyl group, and heteroatom-containing groups thereof. Examples of the substituted or unsubstituted cyclic alkyl groups each optionally containing a heteroatom include a cyclopropyl group, a cyclobutyl group, a cyclopentyl group, a cyclohexyl group, a cycloheptyl group, a cyclooctyl group, an adamanthyl group, a 1-ethylcyclopentyl group, a 1-ethylcyclohexyl group, and heteroatom-containing groups thereof. Examples of the substituted or unsubstituted cyclic ether groups each optionally containing a heteroatom include an oxirane group, an oxetane group, an oxolane group, an oxane group, an oxepane group, an oxocane group, an oxonane group, an oxecane group, an oxete group, an oxole group, a dioxolane group, a dioxane group, a dioxepane group, a dioxecane group, and heteroatom-containing groups thereof. Examples of the substituted or unsubstituted aryl groups each optionally containing a heteroatom include a phenyl group, a tolyl group, a xylyl group, a biphenyl group, a naphthyl group, an anthryl group, a phenanthryl group, and heteroatom-containing groups thereof. Examples of the substituted or unsubstituted aralkyl groups each optionally containing a heteroatom include a benzyl group, a phenethyl group, and heteroatom-containing groups thereof. Examples of the substituted or unsubstituted alkoxy groups each optionally containing a heteroatom include a methoxy group, an ethoxy group, an n-propoxy group, an isopropoxy group, an n-butoxy group, a sec-butoxy group, an isobutoxy group, a t-butoxy group, an n-pentyloxy group, an n-hexyloxy group, and heteroatom-containing groups thereof.

Examples of the substituted or unsubstituted divalent hydrocarbon groups each optionally containing a heteroatom include substituted or unsubstituted alkylene, alkenylene, cycloalkylene, cycloalkylalkylene, arylene, aralkylene, and oxyalkylene groups each optionally containing a heteroatom.

Specific examples of the substituted or unsubstituted alkylene groups each optionally containing a heteroatom include a methylene group, an ethylene group, a trimethylene group, a tetramethylene group, a pentamethylene group, a hexamethylene group, an octylene group, a nonylene group, a decylene group, a 1,2-propylene group, and heteroatom-containing groups thereof. Examples of the substituted or unsubstituted alkenylene groups each optionally containing a heteroatom include a vinylene group, a 1-propenylene group, a 2-propenylene group, and heteroatom-containing groups thereof. Examples of the substituted or unsubstituted cycloalkylene groups each optionally containing a heteroatom include a cyclohexylene group and heteroatom-containing groups thereof. Examples of the substituted or unsubstituted cycloalkylalkylene groups each optionally containing a heteroatom include a cyclohexylmethylene group and heteroatom-containing groups thereof. Examples of the substituted or unsubstituted arylene groups each optionally containing a heteroatom include a phenylene group, a tolylene group, a xylylene group, and heteroatom-containing groups thereof. Examples of the substituted or unsubstituted aralkylene groups each optionally containing a heteroatom include a benzylidene group and heteroatom-containing groups thereof. Examples of the substituted or unsubstituted oxyalkylene groups each optionally containing a heteroatom include an oxyethylene group, an oxypropylene group, an oxybutylene group, an oxytetramethylene group, and heteroatom-containing groups thereof.

The surface of the soda-lime glass substrate having "at least one group represented by -N(R¹)₂ or -N(R²)₃⁺" can be formed by any method capable of introducing at least one group represented by -N(R¹)₂ or -N(R²)₃⁺ to a surface of the substrate. The introduction method may be carried out, for example, by treating a substrate surface with a silane coupling agent containing at least one group represented by -N(R¹)₂ or -N(R²)₃⁺ (silane coupling agent treatment) or by treating a substrate surface with NH₃ gas (NH₃ gas treatment). Silane coupling agent treatment is preferred among these.

Any silane coupling agent containing at least one group represented by -N(R¹)₂ or -N(R²)₃⁺ may be used in the silane coupling agent treatment, and examples include compounds containing at least one group represented by -N(R¹)₂ or -N(R²)₃⁺ and a group represented by -Si(R³)₃, wherein R¹s or R²s are the same as or different from each other and each represent a hydrogen atom or a substituted or unsubstituted hydrocarbon group optionally containing a heteroatom, and R³s are the same as or different from each other and each represent a substituted or unsubstituted hydrocarbon group optionally containing a heteroatom.

R³s in the group represented by -Si(R³)₃ are the same as or different from each other and each represent a substituted or unsubstituted hydrocarbon group optionally containing a heteroatom. Examples of the substituted or unsubstituted hydrocarbon group optionally containing a heteroatom include those described for the substituted or unsubstituted monovalent hydrocarbon groups each optionally containing a heteroatom for R¹ and R². Preferred among these are linear alkyl groups, branched or cyclic alkyl groups, and alkoxy groups. Specific examples of the linear alkyl groups, branched or cyclic alkyl groups, and alkoxy groups include those described for R¹ and R² (e.g., a methyl group, an ethyl group, a methoxy group, an ethoxy group). Preferably, at least one of the R³ groups is an alkoxy group; more preferably, two of the R³ groups are alkoxy groups; still more preferably, three of the R³ groups are alkoxy groups.

Suitable examples of the compounds containing at least one group represented by -N(R¹)₂ or -N(R²)₃⁺ and a group represented by -Si(R³)₃ include compounds represented by the following formulas (A-1) and (A-2).

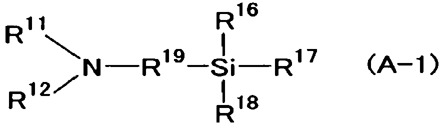

In the formula, R¹¹ and R¹² are the same as or different from each other and each represent a hydrogen atom or a substituted or unsubstituted monovalent hydrocarbon group optionally containing a heteroatom; R¹⁶ to R¹⁸ are the same as or different from each other and each represent a substituted or unsubstituted monovalent hydrocarbon group optionally containing a heteroatom; and R¹⁹ represents a substituted or unsubstituted divalent hydrocarbon group optionally containing a heteroatom.

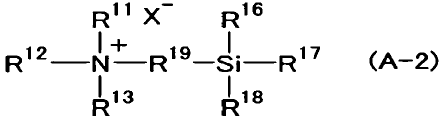

In the formula, R¹¹ to R¹³ are the same as or different from each other and each represent a hydrogen atom or a substituted or unsubstituted monovalent hydrocarbon group optionally containing a heteroatom; R¹⁶ to R¹⁸ are the same as or different from each other and each represent a substituted or unsubstituted monovalent hydrocarbon group optionally containing a heteroatom; R¹⁹ represents a substituted or unsubstituted divalent hydrocarbon group optionally containing a heteroatom; and X represents a halogen.

Examples of the "substituted or unsubstituted monovalent hydrocarbon group optionally containing a heteroatom" for R¹¹, R¹², and R¹³ in formulas (A-1) and (A-2) include those described for the substituted or unsubstituted monovalent hydrocarbon group optionally containing a heteroatom for R¹ and R². In particular, R¹¹, R¹², and R¹³ are each preferably a hydrogen atom or a linear or branched alkyl group optionally containing a heteroatom, more preferably a hydrogen atom, a linear or branched alkyl group, or a group represented by -CₚH₂ₚ-O-C_{q}H_{2q+1} wherein p and q each represent an integer.

Examples of the "substituted or unsubstituted monovalent hydrocarbon group optionally containing a heteroatom" for R¹⁶, R¹⁷, and R¹⁸ in formulas (A-1) and (A-2) include those described for the substituted or unsubstituted monovalent hydrocarbon group optionally containing a heteroatom for R¹ and R². In particular, R¹⁶, R¹⁷, and R¹⁸ are each preferably a substituted or unsubstituted alkoxy group optionally containing a heteroatom, more preferably an alkoxy group, still more preferably a methoxy group or an ethoxy group.

Examples of the "substituted or unsubstituted divalent hydrocarbon group optionally containing a heteroatom" for R¹⁹ in formulas (A-1) and (A-2) include those described for the substituted or unsubstituted divalent hydrocarbon group optionally containing a heteroatom for R¹ and R². In addition to the specific examples of the substituted or unsubstituted divalent hydrocarbon group optionally containing a heteroatom for R¹, examples also include groups represented by -CO-N(R¹¹¹)-(R¹¹²)-wherein R¹¹¹ represents a hydrogen atom or an alkyl group, and R¹¹² represents a group represented by -CₚH₂ₚ- wherein p represents an integer of 1 to 8. In particular, R¹⁹ is preferably a substituted or unsubstituted alkylene group optionally containing a heteroatom or a group represented by -CO-N(R¹¹¹)-(R¹¹²)-, more preferably a methylene group, an ethylene group, a trimethylene group, a tetramethylene group, or a group represented by -CO-N(R¹¹¹)-(R¹¹²)-.

Examples of the halogen for X in formula (A-2) include chlorine (chlorine ion) and bromine (bromine ion).

Other suitable examples of the compounds containing at least one group represented by -N(R¹)₂ or -N(R²)₃⁺ and a group represented by -Si(R²)₃ include compounds represented by the following formula (A-3): 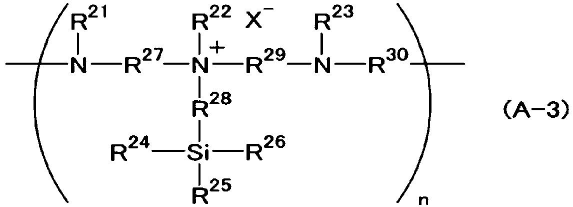 wherein R²¹ to R²³ are the same as or different from each other and each represent a hydrogen atom or a substituted or unsubstituted monovalent hydrocarbon group optionally containing a heteroatom; R²⁴ to R²⁶ are the same as or different from each other and each represent a substituted or unsubstituted monovalent hydrocarbon group optionally containing a heteroatom; R²⁷ to R³⁰ each represent a substituted or unsubstituted divalent hydrocarbon group optionally containing a heteroatom; X represents a halogen; and n represents the number of repetitions.

Examples of the "substituted or unsubstituted monovalent hydrocarbon group optionally containing a heteroatom" for R²¹, R²², and R²³ in formula (A-3) include those described for the substituted or unsubstituted monovalent hydrocarbon group optionally containing a heteroatom for R¹ and R². In particular, R²¹, R²², and R²³ are each preferably a hydrogen atom or a linear or branched alkyl group optionally containing a heteroatom, more preferably a hydrogen atom.

Examples of the "substituted or unsubstituted monovalent hydrocarbon group optionally containing a heteroatom" for R²⁴, R²⁵, and R²⁶ in formula (A-3) include those described for the substituted or unsubstituted monovalent hydrocarbon group optionally containing a heteroatom for R¹ and R². In particular, R²⁴, R²⁵, and R²⁶ are each preferably a substituted or unsubstituted alkoxy group optionally containing a heteroatom, more preferably an alkoxy group, still more preferably a methoxy group or an ethoxy group.

Examples of the "substituted or unsubstituted divalent hydrocarbon group optionally containing a heteroatom" for R²⁷, R²⁸, R²⁹, and R³⁰ in formula (A-3) include those described for the substituted or unsubstituted divalent hydrocarbon group optionally containing a heteroatom for R¹ and R². In particular, R²⁷, R²⁸, R²⁹, and R³⁰ are each preferably a substituted or unsubstituted alkylene group optionally containing a heteroatom, more preferably a methylene group, an ethylene group, a trimethylene group, or a tetramethylene group.

Examples of the halogen for X in formula (A-3) include chlorine (chlorine ion) and bromine (bromine ion).

The degree of polymerization of the structural unit represented by formula (A-3) is not limited and may be appropriately selected in view of the processability (coating properties) of the compound, adhesion to the hydrophilic polymer layer, or other factors.

The number average molecular weight (Mn) of the compounds (polymers) represented by formula (A-3) is preferably 500 to 6000, more preferably 1000 to 4000, still more preferably 1000 to 3000.

Specific examples of the silane coupling agent containing the group represented by -N(R¹)₂ include amino group-containing silane coupling agents such as 3-aminopropyltrimethoxysilane, 3-aminopropyltriethoxysilane, 3-aminopropyltriisopropoxysilane, 3-aminopropylmethyldimethoxysilane, 3-aminopropylmethyldiethoxysilane, 3-(2-aminoethyl)aminopropyltrimethoxysilane, 3-(2-aminoethyl)aminopropylmethyldimethoxysilane, 3-(2-aminoethyl)aminopropyltriethoxysilane, 3-(2-aminoethyl)aminopropylmethyldiethoxysilane, 3-(2-aminoethyl)aminopropyltriisopropoxysilane, 3-(2-(2-aminoethyl)aminoethyl)aminopropyltrimethoxysilane, 3-(6-aminohexyl)aminopropyltrimethoxysilane, 3-(N-ethylamino)-2-methylpropyltrimethoxysilane, N-phenyl-3-aminopropyltrimethoxysilane, N-benzyl-3-aminopropyltrimethoxysilane, N-vinylbenzyl-3-aminopropyltriethoxysilane, N-cyclohexylaminomethyltriethoxysilane, N-cyclohexylaminomethyldiethoxymethylsilane, N-phenylaminomethyltrimethoxysilane, and (2-aminoethyl)aminomethyltrimethoxysilane; and urea group-containing silane coupling agents such as 3-ureidopropyltrimethoxysilane (1-[3-(trimethoxysilyl)propyl]urea), 3-ureidopropyltriethoxysilane, 3-ureidopropyltriisopropoxysilane, 3-ureidopropylmethyldimethoxysilane, 3-ureidopropylmethyldiethoxysilane, ureidomethyltrimethoxysilane, ureidomethyltriethoxysilane, ureidomethyltriisopropoxysilane, ureidomethylmethyldimethoxysilane, ureidomethylmethyldiethoxysilane, 3-[(2-ureidoethyl)amino]propyltrimethoxysilane, 1,3-bis[3-(trimethoxysilyl)propyl]urea, 1,3-bis[3-(triethoxysilyl)propyl]urea, 1-[3-(triethoxysilyl)propyl]-3-phenylurea, 1,3-bis[3-(tripropylsilyl)propyl]urea, 1-[3-(triethoxysilyl)propyl]-3,3-dioctylurea, 1-[3-(triethoxysilyl)propyl]-3,3-dihexadecylurea, 1-[3-(triethoxysilyl)propyl]-3-propylurea, 3-(3-dodecylureido)propyltriethoxysilane, and N-(1-phenylethyl)-N'-[3-(triethoxysilyl)propyl]urea.

Specific examples of the silane coupling agent containing the group represented by -N(R¹)₃⁺ include N-trimethoxysilylpropyl-N,N,N-trimethylammonium chloride, N-trimethoxysilylpropyl-N,N,N-tri-n-butylammonium chloride, octadecyldimethyl(3-trimethoxysilyl-propyl)ammonium chloride, N-(trimethoxysilylethyl)benzyl-N,N,N-trimethylammonium chloride, N,N-didecyl-N-methyl-N-(3-trimethoxysilyl-propyl)ammonium chloride, bis(methoxyethyl)-3-trimethoxysilylpropylammonium chloride, trimethoxysilylpropyl modified (polyethylenimine), and dimethoxymethylsilylpropyl modified (polyethylenimine).

Preferred among the silane coupling agents listed above are 3-aminopropyltriethoxysilane, bis(methoxyethyl)-3-trimethoxysilylpropylammonium chloride, trimethoxysilylpropyl modified (polyethylenimine), and N-trimethoxysilylpropyl-N,N,N-trimethylammonium chloride. Each of these silane coupling agents containing at least one group represented by -N(R¹)₂ or -N(R²)₃⁺ containing at least one group represented by -N(R¹)₂ or -N(R²)₃⁺ may be used alone, or two or more of these may be used in combination.

The silane coupling agent treatment (treating a substrate surface with a silane coupling agent containing at least one group represented by -N(R¹)₂ or -N(R¹)₃⁺) may be carried out by any method that can coat (treat) a substrate surface with the silane coupling agent. Specifically, a substrate having a surface coated (treated) with the silane coupling agent can be prepared by a known method such as by coating (application), spraying, or immersion, performed on a substrate surface, using a silane coupling agent solution or dispersion prepared by dissolving or dispersing the silane coupling agent in a solvent. Known coating, spraying, or immersion methods can be used.

The solvent may be any solvent that can dissolve or disperse the silane coupling agent, and may be appropriately selected according to the silane coupling agent used. The solvent may be water, an organic solvent, or a mixture thereof. Examples of the organic solvent include alcohols such as methanol, ethanol, n-propanol, i-propanol, and methoxypropanol; ketones such as acetone and methyl ethyl ketone; tetrahydrofuran, acetonitrile, ethyl acetate, and toluene.

The concentration of the silane coupling agent solution or dispersion may be appropriately selected according to, for example, the type of the silane coupling agent, the type of the solvent, or the treatment method (coating method, spraying method, or immersion method).

The conditions of the silane coupling agent treatment, such as the temperature and duration of the treatment, may be appropriately selected according to, for example, the type of the silane coupling agent, the type of the solvent, or the treatment method. Still, the substrate surface is preferably treated (retained) at a humidity of 50% or higher during the treatment (e.g., coating, spraying, or immersion) with the silane coupling agent solution or dispersion. Under such treatment conditions, the chemical bond between the substrate surface and the silane coupling agent tends to become strong. The humidity is more preferably 60% or higher, still more preferably 80% or higher. The upper limit of the humidity is not limited, but is preferably, for example, 100% or lower.

When the substrate surface is treated (retained) at a humidity of 50% or higher, the duration and the temperature of the treatment may be appropriately selected, for example, from the standpoint of a strong chemical bond between the substrate surface and the silane coupling agent or from an economic standpoint. For example, the duration of the treatment (retention time) is preferably 1 to 60 hours, more preferably 1 to 20 hours. The treatment temperature (retention temperature) is preferably 20°C to 65°C, more preferably 25°C to 50°C.

Although the substrate having a surface coated (treated) with the silane coupling agent may be prepared as described above, a commercial product may also be used as the substrate. Examples of commercial products treated with a silane coupling agent include MAS coated glass slides, MAS-GP type coated glass slides, Frontier coated glass slides, and APS coated glass slides (all available from Matsunami Glass Ind., Ltd.).

From the standpoint of adhesion of the polymer layer to the substrate surface, the substrate surface containing at least one group represented by -N(R¹)₂ or -N(R²)₃⁺ prepared as described above or by other methods preferably at least partially (partially or entirely) has a water contact angle of 50° to 120°, more preferably 70° to 100°.

The soda-lime glass has a bubble contact angle in water of 100° or greater and 120° or less.

The soda-lime glass with a bubble contact angle in water of 100° or greater and 120° or less may be appropriately selected from commercial products, for example.

The bubble contact angle in water of the soda-lime glass is more preferably 103° or greater and more preferably 117° or less.

Herein, the term "bubble contact angle in water" refers to a value measured by putting a bubble (air) onto the substrate surface in water and measuring a contact angle between the bubble and the substrate surface. In the measurement, the temperature of the water may be room temperature (20°C), and the volume of the bubble may be 2 µL.

The glass substrate formed of the soda-lime glass may have any thickness. The average thickness is preferably 100 µm or more and 5000 µm or less, more preferably 500 µm or more and 3000 µm or less. The average thickness is an average of the thicknesses measured at ten arbitrary points using a micrometer.

The polymer-coated glass substrate includes one or two or more polymer layers formed on the surface of the glass substrate. The one or two or more polymer layers may include one polymer layer or may include two or more polymer layers. In particular, in the case where the soda-lime glass substrate has a surface having at least one group represented by -N(R¹)₂ or -N(R²)₃⁺ wherein R¹s or R²s are the same as or different from each other and each represent a hydrogen atom or a substituted or unsubstituted hydrocarbon group optionally containing a heteroatom, the polymer-coated glass substrate preferably includes two or more polymer layers.

Known polymers may be appropriately used as the polymers forming the one or two or more polymer layers. Examples of the polymers include homopolymers of a single monomer and copolymers of two or more monomers. The polymers are suitably polymers with hydrophilicity (hydrophilic polymers). Each of the polymers may be used alone, or two or more of the polymers may be used in combination.

The polymers can be produced by known methods. For example, such a polymer may be synthesized by polymerizing a monomer of the polymer by a known method using a solution of the monomer. Non-limiting examples of the solvent of the monomer solution include those described later. Toluene and methanol are preferred among these.

To better achieve the advantageous effects, in the polymer-coated glass substrate, the outermost layer (polymer layer located on the outermost side (polymer layer defining the surface)) among the one or two or more polymer layers is desirably formed of hydrophilic polymers.

Examples of the hydrophilic polymers include homopolymers or copolymers of one or two or more hydrophilic monomers, and copolymers of one or two or more hydrophilic monomers and one or two or more additional monomers. Each of these may be used alone, or two or more of these may be used in combination.

Non-limiting examples of the hydrophilic monomers include various monomers containing hydrophilic groups. Examples of the hydrophilic groups include known hydrophilic groups such as an amide group, a sulfuric acid group, a sulfonic acid group, a carboxylic acid group, a hydroxy group, an amino group, and an oxyethylene group.

Specific examples of the hydrophilic monomers include (meth)acrylic acids, (meth)acrylic acid esters (e.g., alkoxyalkyl (meth)acrylates such as methoxyethyl (meth)acrylates, and hydroxyalkyl (meth)acrylates such as hydroxyethyl (meth)acrylates), (meth)acrylamides, and (meth)acrylamide derivatives containing cyclic groups (e.g., (meth)acryloylmorpholines). Preferred among these are (meth)acrylic acids, (meth)acrylic acid esters, and alkoxyalkyl (meth)acrylates, with alkoxyalkyl (meth)acrylates being more preferred, with 2-methoxyethyl acrylate being particularly preferred. Each of these may be used alone, or two or more of these may be used in combination.

The additional monomers may be appropriately selected from monomers that do not inhibit the advantageous effect of the hydrophilic polymers. Specific examples of the additional monomers include aromatic monomers such as styrene, vinyl acetate, and N-isopropylacrylamide which can impart temperature responsiveness. Each of these may be used alone, or two or more of these may be used in combination.

Specific examples of the homopolymers and copolymers include homopolymers formed from single hydrophilic monomers, such as polyacrylic acids, polyacrylic acid esters, polymethacrylic acids, polymethacrylic acid esters, polyacryloylmorpholines, polymethacryloylmorpholines, polyacrylamides, polymethacrylamides, polyalkoxyalkyl acrylates, and polyalkoxyalkyl methacrylates; copolymers formed from two or more hydrophilic monomers listed above; and copolymers formed from one or two or more hydrophilic monomers listed above and one or two or more additional monomers listed above. Each of these hydrophilic polymers may be used alone, or two or more of these may be used in combination.

Preferred among the hydrophilic polymers are polymers represented by the following formula (I). Each of these may be used alone, or two or more of these may be used in combination.

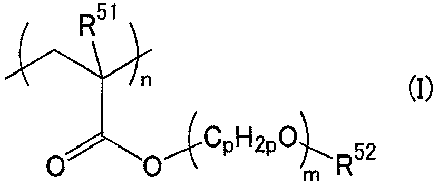

In the formula, R⁵¹ represents a hydrogen atom or a methyl group; R⁵² represents an alkyl group; p represents 1 to 8; m represents 1 to 5; and n represents the number of repetitions.

Suitable examples of the polymers represented by formula (I) include polymers represented by the following formula (I-1). Each of these may be used alone, or two or more of these may be used in combination.

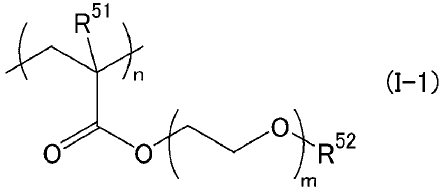

In the formula, R⁵¹ represents a hydrogen atom or a methyl group; R⁵² represents an alkyl group; m represents 1 to 5; and n represents the number of repetitions.

The number of carbon atoms of the alkyl group for R⁵² is preferably 1 to 10, more preferably 1 to 5. In particular, R⁵² is particularly preferably a methyl group or an ethyl group. The symbol p is preferably 1 to 5, more preferably 1 to 3. The symbol m is preferably 1 to 3. The symbol n (the number of repeating units) is preferably 15 to 1500, more preferably 40 to 1200.

Other suitable examples of the hydrophilic polymers include copolymers of compounds represented by the following formula (II) and additional monomers. Each of these may be used alone, or two or more of these may be used in combination.

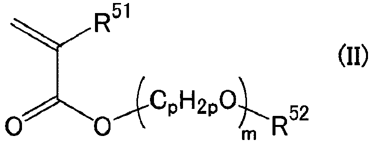

In the formula, R⁵¹, R⁵², p, and m are as defined above.

Suitable examples of the compounds represented by formula (II) include compounds represented by the following formula (II-1). Each of these may be used alone, or two or more of these may be used in combination.

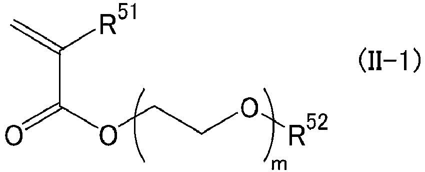

In the formula, R⁵¹, R⁵², and m are as defined above.

To better achieve the advantageous effects, the hydrophilic polymers represented by formula (I) are preferred among the above-mentioned hydrophilic polymers, with the hydrophilic polymers represented by formula (I-1) being particularly preferred.

To better achieve the advantageous effects, the number average molecular weight (Mn) of each polymer forming the outermost layer among the one or two or more polymer layers is preferably 8000 to 150000, more preferably 10000 to 60000, still more preferably 10000 to 39000. A similar number average molecular weight (Mn) is also desirable when the polymer is a hydrophilic polymer.

Herein, the number average molecular weight (Mn) and the weight average molecular weight (Mw) can be determined by gel permeation chromatography (GPC) (GPC-8000 series available from Tosoh Corporation, detector: differential refractometer, column: TSKgel SuperMultipore HZ-M available from Tosoh Corporation) and calibrated with polystyrene standards.

The total thickness of the one or two or more polymer layers (the total thickness of a polymer layer including one or two or more polymer layers) is preferably 10 to 4000 nm, more preferably 30 to 2000 nm, still more preferably 50 to 500 nm. The total thickness within the ranges is expected to lead to favorably low adsorption of proteins and cells and excellent selective adsorption of cancer cells. In the case where the outermost layer is a hydrophilic polymer layer (a layer formed of hydrophilic polymers), the total thickness (total film thickness) is also desirably within the above ranges.

Herein, the total thickness of the polymer layers can be measured using a transmission electron microscope (TEM).

The surface of the polymer layer (the surface of the outermost layer among the one or two or more polymer layers in the polymer-coated glass substrate) preferably at least partially (partially or entirely) has a water contact angle of 65° or less, more preferably 60° or less, still more preferably 55° or less. The water contact angle is preferably 25° or greater, more preferably 33° or greater.

The polymer-coated glass substrate may suitably include a polymer layer formed of a blend of polymers with different molecular weights (hereinafter, also referred to as "blend polymer layer") on the surface of the soda-lime glass substrate. The hydrophilic polymers with different molecular weights may include two polymers which have the same structural unit and different molecular weights (for example, a blend of polymers including poly(2-methoxyethyl acrylate) having a number average molecular weight of 16000 and poly(2-methoxyethyl acrylate) having a number average molecular weight of 80000) or may include two polymers which have different structural units and different molecular weights (for example, a blend of polymers including poly(2-methoxyethyl acrylate) having a number average molecular weight of 16000 and poly(acryloylmorpholine) having a number average molecular weight of 80000).

In particular, the blend polymer layer is desirably formed of polymers having the same structural unit and different molecular weights
The polymers forming the blend polymer layer preferably include polymers with different molecular weights represented by formula (I). The polymers with different molecular weights represented by formula (I) may include two or more polymers which have the same structural unit and different molecular weights (for example, the polymers include poly(2-methoxyethyl acrylate) having a number average molecular weight of 16000 and poly(2-methoxyethyl acrylate) having a number average molecular weight of 80000) or may include two or more polymers which have different structural units and different molecular weights (for example, the polymers include poly(2-methoxyethyl acrylate) having a number average molecular weight of 16000 and poly(2-ethoxyethyl methacrylate) having a number average molecular weight of 80000). One type or two or more types of the polymers represented by formula (I) may be used.

Non-limiting examples of the molecular weight of the polymers with different molecular weights include number average molecular weight (Mn) and weight average molecular weight (Mw). A blend of polymers with different Mn may suitably be used.

In the case of the blend of polymers with different Mn, the blend preferably includes polymers with a difference in Mn of 30000 or more and more preferably includes hydrophilic polymers with a difference in Mn of 50000 or more.

The blend of polymers with different molecular weights preferably includes polymers with low molecular weights having a number average molecular weight (Mn) of 5000 or more and 25000 or less. The polymers with low molecular weights each have Mn of preferably 10000 or more, more preferably 14000 or more and preferably 23000 or less.

The blend of polymers with different molecular weights desirably includes polymers with high molecular weights having a number average molecular weight (Mn) of 35000 or more and 200000 or less. The polymers with high molecular weights each have Mn of preferably 40000 or more, more preferably 50000 or more, and preferably 150000 or less, more preferably 130000 or less.

A polymer-coated glass substrate in which a polymer layer is formed entirely or partially on the surface of a glass substrate can be prepared by a known technique, such as 1) by dissolving or dispersing a polymer in a solvent to prepare a polymer solution or dispersion, introducing the polymer solution or dispersion onto the surface of the glass substrate (e.g., the recess of the substrate), and retaining and drying the substrate for a predetermined time, or 2) by applying (spraying) the polymer solution or dispersion to the surface of the glass substrate and optionally drying the substrate. Then, the polymer-coated glass substrate may be combined with other components as needed to produce a device capable of, for example, capturing, culturing, or analyzing specific cells.

Conventionally known materials or methods are applicable as the solvent, introduction method, application (spraying) method, and other materials and methods.

The duration of retention or drying in the technique 1) or 2) may be appropriately selected depending on the size of the substrate, the type of liquid introduced, or other factors. The duration of retention is preferably 10 seconds to 10 hours, more preferably 1 minute to 5 hours, still more preferably 5 minutes to 2 hours. The drying is preferably performed at room temperature (about 23°C) to 80°C, more preferably at room temperature to 60°C. Moreover, the drying may be carried out under reduced pressure. Furthermore, after a certain retention time, the excess polymer solution or dispersion may be discharged as appropriate before drying.

The solvent may be any solvent that can dissolve a polymer and may be appropriately selected depending on the polymer used. For example, the solvent may be water, an organic solvent, or a mixture thereof. Examples of the organic solvent include alcohols such as methanol, ethanol, n-propanol, i-propanol, and methoxypropanol; ketones such as acetone and methyl ethyl ketone; tetrahydrofuran, acetonitrile, ethyl acetate, and toluene.

The concentration of the polymer solution or dispersion is not limited and may be appropriately selected in view of, for example, introduction, coating properties, spraying properties, or productivity. The concentration of the polymers in the polymer solution or dispersion (100% by mass) is preferably 0.1 to 10.0% by mass, more preferably 0.2 to 5.0% by mass.

In the polymer-coated glass substrate, the outermost layer among the one or two or more polymer layers formed on the surface of the glass substrate has a surface with an elastic modulus in water or an aqueous solution of 1.20 MPa or lower. From the standpoint of inhibiting the adsorption of normal cells such as blood cells and achieving selective adsorption of specific cells such as cancer cells, the elastic modulus is preferably 1.00 MPa or lower, more preferably 0.85 MPa or lower, still more preferably 0.75 MPa or lower. The lower limit is preferably 0.02 MPa or higher, more preferably 0.04 MPa or higher, still more preferably 0.45 MPa or higher, particularly preferably 0.50 MPa or higher.

The elastic modulus in water or an aqueous solution of the surface of the outermost layer can be controlled by changing the molecular weights of the polymers forming the outermost layer or the film thickness. Specifically, the elastic modulus tends to increase as the molecular weights of the polymers increase or as the film thickness of the polymer layer increases.

The elastic modulus in water or an aqueous solution of the surface of the outermost layer can also be controlled through prior surface treatment, such as primer treatment or silane coupling agent treatment, of the glass substrate.

Herein, the elastic modulus in water or an aqueous solution of the surface of the outermost layer refers to an elastic modulus in water or an aqueous solution measured using an atomic force microscope (AFM), unless otherwise stated.

An atomic force microscope (AFM) is a type of scanning probe microscope which detects the force acting between the atoms on the sample and the atoms on the probe. The probe is attached to the tip of a cantilever (cantilever spring). The force (amount of deflection) acting on the cantilever can be measured while changing the distance between the sample and the probe to obtain a curve (force curve) plotting the relationships between them. Analysis of the force curve can determine the elastic modulus (hardness) of the surface of the sample at the nano scale. The method for determining the elastic modulus of the surface of the sample by the force curve analysis is known to those skilled in the art. The elastic modulus can be determined by such a known method.

For example, the elastic modulus may be calculated from the force curve by fitting the force curve according to the Johnson-Kendall-Roberts (JKR) theory to calculate the elastic modulus. According to the JKR theory, the force F applied to the cantilever and the amount of deformation δ of the sample are expressed by the following equations (1) and (2) with w representing the adhesion energy. $F = \frac{{a}^{3}}{R} - \sqrt{6 {πKwa}^{3}}$ (1) $δ = \frac{{a}^{2}}{3 R} + \frac{2 F}{3 aK}$ (2)

In the equations, a denotes the radius of a contact line between the probe and the sample, R denotes the radius of curvature of the probe tip, and K denotes the elastic coefficient.

The elastic modulus can be determined from the F-δ curve obtained by the force curve analysis and the fitting using equations (1) and (2).

Here, the elastic modulus in water or an aqueous solution of the surface of the outermost layer specifically refers to the value measured as follows.

The elastic modulus in water or an aqueous solution of the surface of the outermost layer can be measured with an AFM by dropping water or an aqueous solution on the surface of the outermost layer of the sample to form a droplet (convex meniscus).

Suitable examples of the aqueous solution include phosphate buffered saline (PBS).

Then, the elastic modulus of the sample (the surface of the outermost layer) may be determined, for example, by scanning the surface of the outermost layer of the sample within a predetermined range to acquire force curves at multiple points within the predetermined range, determining the elastic moduli from the force curves, and calculating the average of the elastic moduli.

The polymer-coated glass substrate produced by the above method includes a glass substrate including a soda-lime glass with a bubble contact angle in water of 100° or greater and 120° or less, and one or two or more polymer layers formed on the surface of the glass substrate. Additionally, the outermost layer among the polymer layers has a soft surface with an elastic modulus in water or an aqueous solution of 1.20 MPa or lower. Therefore, normal cells such as blood cells are less likely to adsorb to the polymer-coated glass substrate, while specific cells such as cancer cells, stem cells, or T cells highly adsorb to the polymer-coated glass substrate. Thus, the polymer-coated glass substrate is excellent in selective adsorption of specific cells.

### EXAMPLES

The present invention will be specifically described below with reference to examples, but the present invention is not limited to the examples.

### <Example 1>

### (Preparation of polymer)

Using a solution of azobisisobutyronitrile (AIBN) in toluene (12.5 mg/mL), 2-methoxyethyl acrylate (2.5 wt% in toluene) was thermally polymerized at 60°C for seven hours to prepare poly(2-methoxyethyl acrylate) (PMEA).

An amount of 80 µL of a 0.25 wt% solution of the PMEA in methanol was introduced into a chamber slide (two-chamber type, available from Matsunami Glass Ind., Ltd., uncoated, made of soda-lime glass, average thickness 1300 um, bubble contact angle in water 109.9°), immediately followed by vacuum drying (coating) in an oven at 50°C for 20 minutes to prepare a polymer-coated glass substrate.

### <Example 2>

### (Preparation of polymer)

Using a solution of azobisisobutyronitrile (AIBN) in toluene (12.5 mg/mL), 2-methoxyethyl acrylate (2.5 wt% in toluene) was thermally polymerized at 60°C for seven hours to prepare poly(2-methoxyethyl acrylate) (PMEA).

An amount of 145 µL of a 0.155 wt% solution of the PMEA in methanol was introduced into a chamber slide (two-chamber type, available from Matsunami Glass Ind., Ltd., uncoated, made of soda-lime glass, average thickness 1300 um, bubble contact angle in water 109.9°), immediately followed by drying (coating) at normal pressure in an oven at 60°C for 15 minutes to prepare a polymer-coated glass substrate.

### <Example 3>

### (Preparation of polymer)

Using a solution of azobisisobutyronitrile (AIBN) in toluene (12.5 mg/mL), 2-methoxyethyl acrylate (2.5 wt% in toluene) was thermally polymerized at 60°C for seven hours to prepare poly(2-methoxyethyl acrylate) (PMEA).

An amount of 90 µL of a 0.50 wt% solution of the PMEA in methanol was introduced into a chamber slide (two-chamber type, available from Matsunami Glass Ind., Ltd., uncoated, made of soda-lime glass, average thickness 1300 um, bubble contact angle in water 109.9°), immediately followed by vacuum drying (coating) in an oven at 50°C for 20 minutes to prepare a polymer-coated glass substrate.

### <Comparative Example 1>

### (Preparation of polymer)

Using a solution of azobisisobutyronitrile (AIBN) in methanol (12.5 mg/mL), 2-methoxyethyl acrylate (5.0 wt% in methanol) was thermally polymerized at 60°C for seven hours to prepare poly(2-methoxyethyl acrylate) (PMEA).

An amount of 145 µL of a 0.155 wt% solution of the PMEA in methanol was introduced into a chamber slide (two-chamber type, available from Matsunami Glass Ind., Ltd., uncoated, made of soda-lime glass, average thickness 1300 um, bubble contact angle in water 109.9°), immediately followed by vacuum drying (coating) in an oven at 50°C for 20 minutes to prepare a polymer-coated glass substrate.

### <Example 4>

### (Production of polymer 1)

Using a solution of azobisisobutyronitrile (AIBN) in methanol (1.25 mg/ml), 2-methoxyethyl acrylate (50 wt% in methanol) was thermally polymerized at 60°C for seven hours to produce poly(2-methoxyethyl acrylate) (PMEA, Mn: 80000).

### (Production of polymer 2)

Using a solution of azobisisobutyronitrile (AIBN) in toluene (1.25 mg/ml), 2-methoxyethyl acrylate (25 wt% in toluene) was thermally polymerized at 60°C for seven hours to produce poly(2-methoxyethyl acrylate) (PMEA, Mn: 16000).

An amount of 80 ul of a 0.0375% solution of the PMEA (Mn: 80000) produced in the production of polymer 1 in methanol was introduced into one well of a chamber slide (two-well type, available from Matsunami Glass Ind., Ltd., uncoated, bottom face: soda-lime glass, bubble contact angle in water 109.9°, average thickness: 1.35 µm), immediately followed by vacuum drying in an oven at 40°C for five minutes.

After cooling to room temperature, 90 ul of a 0.16% solution of the PMEA (Mn: 16000) produced in the production of polymer 2 in methanol was introduced onto the PMEA coating layer, immediately followed by vacuum drying in an oven at 40°C for five minutes to prepare a polymer-coated glass substrate.

### <Example 5>

An amount of 80 ul of a 0.0375% solution of the PMEA (Mn: 80000) produced in the production of polymer 1 in methanol was introduced into one well of a chamber slide (two-well type, available from Matsunami Glass Ind., Ltd., uncoated, bottom face: soda-lime glass, bubble contact angle in water 109.9°, average thickness: 1.35 µm), immediately followed by vacuum drying in an oven at 40°C for five minutes.

After cooling to room temperature, 90 ul of a 0.19% solution of the PMEA (Mn: 16000) produced in the production of polymer 2 in methanol was introduced onto the PMEA coating layer, immediately followed by vacuum drying in an oven at 40°C for five minutes to prepare a polymer-coated glass substrate.

### <Example 6>

An amount of 80 ul of a 0.0375% solution of the PMEA (Mn: 80000) produced in the production of polymer 1 in methanol was introduced into one well of a chamber slide (two-well type, available from Matsunami Glass Ind., Ltd., uncoated, bottom face: soda-lime glass, bubble contact angle in water 109.9°, average thickness: 1.35 µm), immediately followed by vacuum drying in an oven at 40°C for five minutes.

After cooling to room temperature, 90 ul of a 0.23% solution of the PMEA (Mn: 16000) produced in the production of polymer 2 in methanol was introduced onto the PMEA coating layer, immediately followed by vacuum drying in an oven at 40°C for five minutes to prepare a polymer-coated glass substrate.

### <Example 7>

An amount of 80 ul of a 0.0375% solution of the PMEA (Mn: 80000) produced in the production of polymer 1 in methanol was introduced into one well of a chamber slide with NH₃⁺ groups having been introduced onto its surface (two-well type, available from Matsunami Glass Ind., Ltd., MAS-coated, bottom face: soda-lime glass, bubble contact angle in water 109.9°, average thickness: 1.35 µm), immediately followed by vacuum drying in an oven at 40°C for five minutes.

After cooling to room temperature, 90 ul of a 0.19% solution of the PMEA (Mn: 16000) produced in the production of polymer 2 in methanol was introduced onto the PMEA coating layer, immediately followed by vacuum drying in an oven at 40°C for five minutes to prepare a polymer-coated glass substrate.

### <Example 8>

An amount of 80 ul of a 0.0750% solution of the PMEA (Mn: 80000) produced in the production of polymer 1 in methanol was introduced into one well of a chamber slide with NH₃⁺ groups having been introduced onto its surface (two-well type, available from Matsunami Glass Ind., Ltd., MAS-coated, bottom face: soda-lime glass, bubble contact angle in water 109.9°, average thickness: 1.35 µm), immediately followed by vacuum drying in an oven at 40°C for five minutes.

After cooling to room temperature, 90 ul of a 0.19% solution of the PMEA (Mn: 16000) produced in the production of polymer 2 in methanol was introduced onto the PMEA coating layer, immediately followed by vacuum drying in an oven at 40°C for five minutes to prepare a polymer-coated glass substrate.

### <Example 9>

An amount of 640 µl of a 0.0375 wt% solution of the PMEA (Mn: 80000) produced in the production of polymer 1 in methanol (the methanol solution was warmed to 40°C) and 720 µl of a 0.19 wt% solution of the PMEA (Mn: 16000) produced in the production of polymer 2 in methanol (the methanol solution was warmed to 40°C) were mixed. Then, 85 µl of the resulting mixed solution was introduced into one well of a chamber slide (two-well type, available from Matsunami Glass Ind., Ltd., uncoated, bottom face: soda-lime glass, bubble contact angle in water 109.9°, average thickness: 1.35 mm), immediately followed by vacuum drying in an oven at 40°C for five minutes.

After cooling to room temperature, 85 µl of the mixed solution was introduced onto the PMEA coating layer, immediately followed by vacuum drying in an oven at 50°C for 20 minutes to prepare a polymer-coated glass substrate.

For the polymer-coated glass substrates prepared in the examples and comparative example, the thickness of the polymer layer and the elastic modulus in an aqueous phosphate buffered solution (PBS) measured using an AFM were determined as described below. Table 1 shows the results.

### [Thickness of polymer layer]

The thickness (film thickness) of each polymer layer formed in the polymer-coated glass substrate was measured with TEM at an acceleration voltage of 200 kV (JEOL Ltd., JEM-2800).

### [Measurement of elastic modulus in phosphate buffer saline using AFM]

Phosphate buffered saline was dropped on the surface of the polymer layer of the polymer-coated glass substrate to form a droplet (convex meniscus). The elastic modulus was measured by the following method using the device (AFM) described below. The determined elastic modulus was defined as the elastic modulus measured in water or an aqueous solution.

In the measurement of the elastic modulus, the obtained force curve was analyzed based on the JKR contact theory to determine the elastic modulus.

### <Method for measuring elastic modulus>

Device (AFM): Jupiter XR available from Oxford Instruments
Measurement mode: AFM force curve mapping
Cantilever: material Si, radius of curvature R of tip = 150 nm, spring constant 0.67 N/m
Measurement range: scan of 20 µm × 20 µm, calculation of elastic modulus by 2-point JKR method
Scanning rate: 1 Hz
Measurement environment: in PBS
Measurement temperature: 23°C

**[Table 1]**

| | Example 1 | Example 2 | Example 3 | Comparative Example 1 |
|---|---|---|---|---|
| Mn of hydrophilic polymers forming polymer layer (one layer) | 15689 | 15689 | 15689 | 80881 |
| Mw of hydrophilic polymers forming polymer layer (one layer) | 64185 | 64185 | 64185 | 405860 |
| Thickness of polymer layer (one layer) (nm) | 165 | 150 | 345 | 160 |
| Elastic modulus in PBS measured using AFM (MPa) | 0.0572 | 0.0737 | 0.0366 | 2.02 |

**[Table 2]**

| | Example 4 | Example 5 | Example 6 | Example 7 | Example 8 | Example 9 |
|---|---|---|---|---|---|---|
| Soda-lime glass substrate | Uncoated | Uncoated | Uncoated | MAS-coated | MAS-coated | Uncoated |
| Mn of hydrophilic polymers forming polymer layer (innermost layer) | 80000 | 80000 | 80000 | 80000 | 80000 | 80000/16000 |
| Mn of hydrophilic polymers forming polymer layer (outermost layer) | 16000 | 16000 | 16000 | 16000 | 16000 | 80000/16000 |
| Thickness of polymer layer (innermost layer) (nm) | 40 | 40 | 40 | 38 | 79 | 135 |
| Thickness of polymer layer (outermost layer) (nm) | 90 | 107 | 140 | 110 | 108 | 140 |
| Elastic modulus in PBS measured using AFM (MPa) | 0.186 | 0.111 | 0.084 | 0.061 | 1.66 | 0.07 |

In Comparative Example 1, the elastic modulus is 2 MPa or higher. Thus, the adsorption of specific cells such as cancer cells is considered to be reduced. Moreover, leukocyte adsorption is considered to be high.

In contrast, in Example 3, the elastic modulus is 1.20 MPa or lower. Compared to Comparative Example 1, the adsorption of specific cells such as cancer cells is considered to be enhanced. Moreover, leukocyte adsorption is considered to be lower than that in Comparative Example 1.

In Example 1, the elastic modulus is about 0.057 MPa. The adsorption of specific cells such as cancer cells is considered to be enhanced compared to Example 3 in which the elastic modulus is lower than 0.04 MPa. Moreover, leukocyte adsorption is considered to be lower than that in Example 3.

In Example 2, the elastic modulus is about 0.07 MPa. Compared to Example 1, the adsorption of specific cells such as cancer cells is considered to be enhanced. Moreover, leukocyte adsorption is considered to be lower than that in Example 1.

The elastic modulus is low in Examples 4 to 6 in which two layers formed of polymers with different molecular weights are formed on the surface of an uncoated soda-lime glass, in Examples 7 and 8 in which two layers formed of polymers with different molecular weights are formed on the NH₃⁺ group-introduced surface of a soda-lime glass, and in Example 9 in which two layers each formed of a blend of polymers with different molecular weights are formed on the surface of an uncoated soda-lime glass. In these examples, the adsorption of specific cells such as cancer cells is considered to be enhanced. Moreover, leukocyte adsorption is considered to be reduced.

The present invention (1) is a polymer-coated glass substrate, including:
a glass substrate; and
one or two or more polymer layers formed on a surface of the glass substrate,
the glass being a soda-lime glass with a bubble contact angle in water of 100° or greater and 120° or less,
an outermost layer among the one or two or more polymer layers having a surface with an elastic modulus in water or an aqueous solution of 1.20 MPa or lower.

The present invention (2) is the polymer-coated glass substrate according to the present invention (1),
wherein the elastic modulus is 0.85 MPa or lower.

The present invention (3) is the polymer-coated glass substrate according to the present invention (1),
wherein the elastic modulus is 0.75 MPa or lower.

The present invention (4) is the polymer-coated glass substrate according to the present invention (1),
wherein the elastic modulus is 0.04 MPa or higher.

The present invention (5) is the polymer-coated glass substrate according to a combination with any one of the present inventions (1) to (4),
wherein the outermost layer is formed of a polymer represented by the following formula (I): 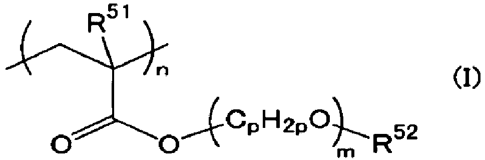 wherein R⁵¹ represents a hydrogen atom or a methyl group; R⁵² represents an alkyl group; p represents 1 to 8; m represents 1 to 5; and n represents the number of repetitions.

The present invention (6) is the polymer-coated glass substrate according to a combination with any one of the present inventions (1) to (4),
wherein the outermost layer is formed of a polymer represented by the following formula (I-1): 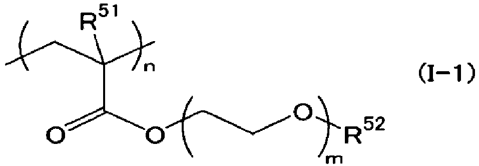 wherein R⁵¹ represents a hydrogen atom or a methyl group; R⁵² represents an alkyl group; m represents 1 to 5; and n represents the number of repetitions.

The present invention (7) is the polymer-coated glass substrate according to a combination with any one of the present inventions (1) to (4),
wherein the outermost layer is formed of a copolymer of a compound represented by the following formula (II) and an additional monomer: 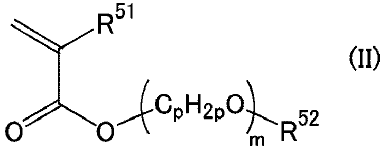 wherein R⁵¹ represents a hydrogen atom or a methyl group; R⁵² represents an alkyl group; p represents 1 to 8; and m represents 1 to 5.

The present invention (8) is the polymer-coated glass substrate according to a combination with any one of the present inventions (1) to (4),
wherein the outermost layer is formed of a copolymer of a compound represented by the following formula (II-1) and an additional monomer: 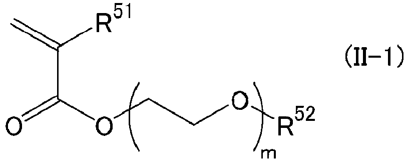 wherein R⁵¹ represents a hydrogen atom or a methyl group; R⁵² represents an alkyl group; and m represents 1 to 5.

The present invention (9) is the polymer-coated glass substrate according to a combination with any one of the present inventions (5) to (8),
wherein the polymer forming the outermost layer has a number average molecular weight of 10000 to 60000.

The present invention (10) is the polymer-coated glass substrate according to a combination with any one of the present inventions (1) to (9),
wherein a total thickness of the one or two or more polymer layers is 10 to 4000 nm.

The present invention (11) is the polymer-coated glass substrate according to a combination with any one of the present inventions (1) to (10),
wherein the elastic modulus is measured with an atomic force microscope.

The present invention (12) is the polymer-coated glass substrate according to a combination with any one of the present inventions (1) to (11),
wherein the one or two or more polymer layers include two or more polymer layers, and
the glass substrate has a surface having at least one group represented by - N(R¹)₂ or -N(R²)₃⁺ wherein R¹s or R²s are the same as or different from each other and each represent a hydrogen atom or a substituted or unsubstituted hydrocarbon group optionally containing a heteroatom.

The present invention (13) is the polymer-coated glass substrate according to a combination with any one of the present inventions (1) to (12),
wherein the outermost layer is formed of a blend of hydrophilic polymers with different molecular weights.

## Claims

1. A polymer-coated glass substrate, comprising:
a glass substrate; and
one or two or more polymer layers formed on a surface of the glass substrate,
the glass being a soda-lime glass with a bubble contact angle in water of 100° or greater and 120° or less,
an outermost layer among the one or two or more polymer layers having a surface with an elastic modulus in water or an aqueous solution of 1.20 MPa or lower.

2. The polymer-coated glass substrate according to claim 1,
wherein the elastic modulus is 0.85 MPa or lower.

3. The polymer-coated glass substrate according to claim 1,
wherein the elastic modulus is 0.75 MPa or lower.

4. The polymer-coated glass substrate according to claim 1,
wherein the elastic modulus is 0.04 MPa or higher.

5. The polymer-coated glass substrate according to any one of claims 1 to 4,
wherein the outermost layer is formed of a polymer represented by the following formula (I): 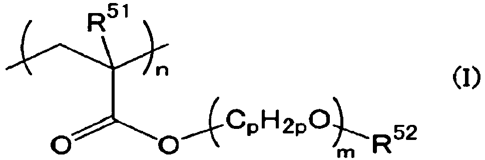 wherein R⁵¹ represents a hydrogen atom or a methyl group; R⁵² represents an alkyl group; p represents 1 to 8; m represents 1 to 5; and n represents the number of repetitions.

6. The polymer-coated glass substrate according to any one of claims 1 to 4,
wherein the outermost layer is formed of a polymer represented by the following formula (I-1): 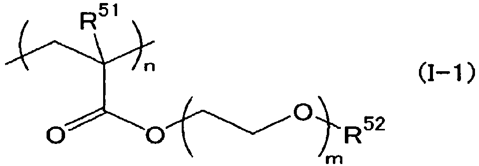 wherein R⁵¹ represents a hydrogen atom or a methyl group; R⁵² represents an alkyl group; m represents 1 to 5; and n represents the number of repetitions.

7. The polymer-coated glass substrate according to any one of claims 1 to 4,
wherein the outermost layer is formed of a copolymer of a compound represented by the following formula (II) and an additional monomer: 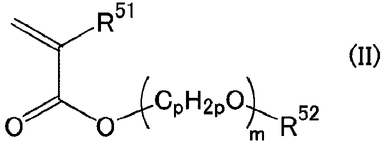 wherein R⁵¹ represents a hydrogen atom or a methyl group; R⁵² represents an alkyl group; p represents 1 to 8; and m represents 1 to 5.

8. The polymer-coated glass substrate according to any one of claims 1 to 4,
wherein the outermost layer is formed of a copolymer of a compound represented by the following formula (II-1) and an additional monomer: 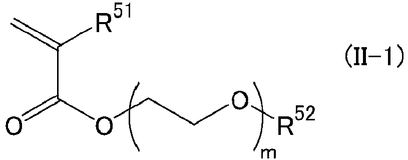 wherein R⁵¹ represents a hydrogen atom or a methyl group; R⁵² represents an alkyl group; and m represents 1 to 5.

9. The polymer-coated glass substrate according to any one of claims 5 to 8,
wherein the polymer forming the outermost layer has a number average molecular weight of 10000 to 60000.

10. The polymer-coated glass substrate according to any one of claims 1 to 9,
wherein a total thickness of the one or two or more polymer layers is 10 to 4000 nm.

11. The polymer-coated glass substrate according to any one of claims 1 to 10,
wherein the elastic modulus is measured with an atomic force microscope.

12. The polymer-coated glass substrate according to any one of claims 1 to 11,
wherein the one or two or more polymer layers include two or more polymer layers, and
the glass substrate has a surface having at least one group represented by - N(R¹)₂ or -N(R²)₃⁺ wherein R¹s or R²s are the same as or different from each other and each represent a hydrogen atom or a substituted or unsubstituted hydrocarbon group optionally containing a heteroatom.

13. The polymer-coated glass substrate according to any one of claims 1 to 12,
wherein the outermost layer is formed of a blend of hydrophilic polymers with different molecular weights.
